(19) Europäisches Patentamt
European Patent Office
Office européen des brevets

(11) Publication number: **0 333 404 A2**

(12) **EUROPEAN PATENT APPLICATION**

(21) Application number: 89302442.2

(22) Date of filing: 13.03.89

(51) Int. Cl.4: **C12P 17/18 , //(C12P17/18, C12R1:465),A61K31:00**

(30) Priority: **14.03.88 GB 8805978**

(43) Date of publication of application: **20.09.89 Bulletin 89/38**

(84) Designated Contracting States: **AT BE CH DE ES FR GB GR IT LI LU NL SE**

(71) Applicant: **AMERICAN CYANAMID COMPANY**
**One Cyanamid Plaza**
**Wayne New Jersey 07470(US)**

(72) Inventor: **Rudd, Brian Arthur Michael**
**72 Exeter Road Rayners Lane**
**Harrow Middlesex(GB)**
Inventor: **Ramsay, Michael Vincent John**
**157 Kings Road**
**South Harrow Middlesex(GB)**

(74) Representative: **Skailes, Humphrey John et al**
**Frank B. Dehn & Co. Imperial House 15-19**
**Kingsway**
**London WC2B 6UZ(GB)**

(54) **Process for the preparation of macrolide compounds.**

(57) S541 (LL-F28249) compounds of formula (I)

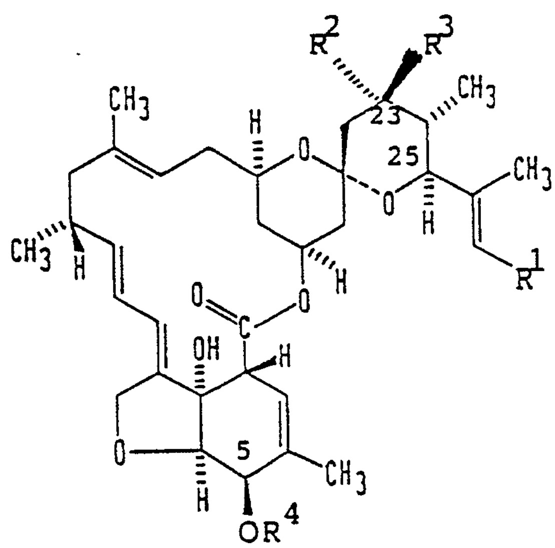

(I)

[wherein $R^1$ is a methyl, ethyl or isopropyl group; $R^2$ is a hydrogen atom or a group $OR^5$ (where $OR^5$ is a hydroxyl group or a substituted hydroxyl group) and $R^3$ is a hydrogen atom, or $R^2$ and $R^3$ together with the carbon atom to which they are attached represent $>C=0$, $>C=CH_2$ or $>C=NOR^6$ (where $R^6$ represents a hydrogen atom, $C_{1-8}$ alkyl group or a $c_{3-8}$ alkenyl group and the group $>C=NOR^6$ is in the E configuration); and $R^4$ is a hydrogen atom or a methyl group] which comprises incubating a corresponding 5-keto compound in a suitable medium in the presence of a microorganism or an enzyme derived therefrom

EP 0 333 404 A2

or a preparation derived from a microorganism containing the enzyme of interest capable of effecting the conversion .

# PROCESS FOR THE PREPARATION OF MACROLIDE COMPOUNDS

This invention relates to a process for the production of macrolide compounds.

United Kingdom Patent Specification 2166436 and European Patent Specification 170006 describe a class of substances, which we have designated Antibiotics S541, which may be prepared by fermentation of Antibiotics S541 producing strains belonging to the species Streptomyces thermoarchaensis and Streptomyces cyaneogriseus noncyanogenus. Antibiotics S541 have antibiotic and, in particular, anti-endoparasitic, anti-ectoparasitic, anti-fungal, insecticidal, nematicidal and acaricidal activity and are of special interest for use in agriculture, horticulture, and animal and human health.

We have now found a new process for the production of Antibiotics S541 compounds and chemical derivatives thereof.

Thus, according to one aspect of the invention we provide a process for the production of compounds of formula (I)

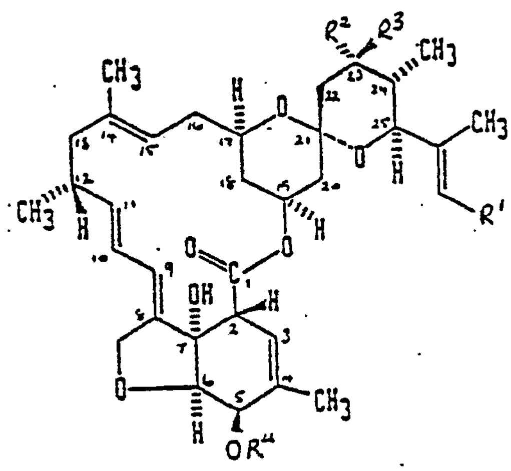

(I)

[wherein $R^1$ is a methyl, ethyl or isopropyl group; $R^2$ is a hydrogen atom or a group $OR^5$ where $OR^5$ is a hydroxyl group or a substituted hydroxyl group having up to 25 carbon atoms) and $R^3$ is a hydrogen atom, or $R^2$ and $R^3$ together with the carbon atom to which they are attached represent $>C=O$, $>C=CH_2$ or $>C=NOR^6$ (where $R^6$ represents a hydrogen atom, a $C_{1-8}$ alkyl group or a $C_{3-8}$ alkenyl group and the group $>C=NOR^6$ is in the E configuration); and $R^4$ is a hydrogen atom or a methyl group] which comprises incubating a compound of formula (II)

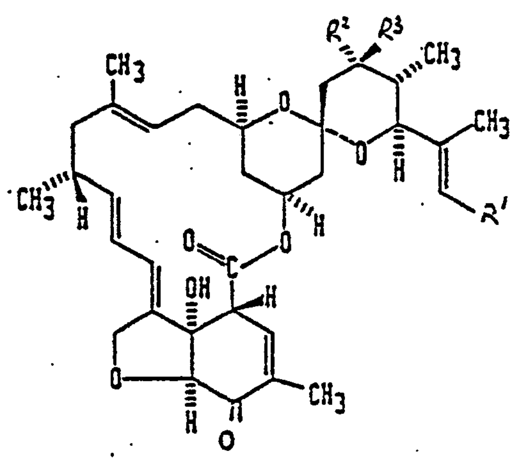

(II)

(wherein $R^1$, $R^2$ and $R^3$ are as defined previously) in a suitable medium in the presence of a microorganism or an enzyme derived therefrom or a preparation derived from a microorganism containing the enzyme of interest capable of effecting the conversion.

Suitable microorganisms and extracts thereof for use in the process according the invention may be identified by preliminary small scale tests designed to demonstrate ability of a microorganism or an extract thereof to convert compounds of formula (II) to compounds of formula (I). The formulation of the compounds of formula (I) may be confirmed by suitable chromatographic analysis (eg high performance liquid chromatography) of the reaction mixture.

We have found microorganisms of the genus Streptomyces and extracts thereof to be particularly suitable for use in the process according to the present invention.

Particular Streptomyces microorganisms for use in the process according to the invention include strains of Streptomyces thermoarchaensis, Streptomyces cyaneogriseus noncyanogenus, Streptomyces avermitilis and Streptomyces hygroscopicus subsp. aureolacrimosus. Particular examples of suitable strains include Streptomyces thermoarchaensis NCIB 12212 and 12213 [deposited 6th March 1986 in the permanent culture collection of the National Collections of Industrial and Marine Bacteria, Torry Research Station, Aberdeen, United Kingdom] and mutants of these strains, Streptomyces avermitilis ATCC 31272 and 31780 and Streptomyces hygroscopicus subsp. aureolacrimosus FERM P 1438.

Mutants of the above strains may arise spontaneously or may be produced by a variety of methods including those described in UK Patent Specification 2166436 and European Patent Specification 170006.

Suitable enzymes for use in the process according to the present invention may be derived from an extremely wide range of sources. The aforementioned Streptomyces microorganisms, however, represent a particularly suitable source of enzymes capable of converting compounds of formula (II) into compounds of formula (I).

In one embodiment of the process according to the invention, the conversion of a compound of formula (II) into a corresponding 5-$OR^4$ compound of formula (I) may be effected by feeding the compound of formula (II) in a suitable solvent into a fermentation medium comprising the aforementioned microorganism in the presence of assimilable sources of carbon, nitrogen and mineral salts.

Assimilable sources of carbon, nitrogen and minerals may be provided by either simple or complex nutrients. Sources of carbon will generally include glucose, maltose, starch, glycerol, molasses, dextrin, lactose, sucrose, fructose, carboxylic acids, amino acids, glycerides, alcohols, alkanes and vegetable oils. Sources of carbon will generally comprise from 0.5 to 10% by weight of the fermentation medium.

Sources of nitrogen will generally include soya bean meal, corn steep liquors, distillers solubles, yeast extracts, cottonseed meal, peptones, ground nut meal, malt extract, molasses, casein, amino acid mixtures, ammonia (gas or solution), ammonium salts or nitrates. Urea and other amides may also be used. Sources of nitrogen will generally comprise from 0.1 to 10% by weight of the fermentation medium.

Nutrient mineral salts which may be incorporated into the culture medium include the generally used salts capable of yielding sodium, potassium, ammonium, iron, magnesium, zinc, nickel, cobalt manganese, vanadium, chromium, calcium, copper, molybdenum, boron, phosphate, sulphate, chloride and carbonate ions.

An antifoam may be present to control excessive foaming and added at intervals as required.

The compound of formula (II) in a solvent such as a water miscible organic solvent (eg an alcohol such as methanol or propan-2-ol, a diol such as propan-1,2-ol or butan-1,3-ol, a ketone such as acetone, a nitrile such as acetonitrile, an ether such as tetrahydrofuran or dioxan, a substituted amide such as dimethylformamide or a dialkylsulphoxide such as dimethylsulphoxide) may be added at the beginning of the cultivation, or more usually, when the growth of the microorganism is under way, e.g. 2 to 4 days after the start of the cultivation.

Cultivation of the organism will generally be effected at a temperature of from 20 to 50° C, preferably from 25 to 40° C, and will desirably take place with aeration and agitation e.g. by shaking or stirring. The medium may initially be inoculated with a small quantity of a suspension of the sporulated microorganism but in order to avoid a growth lag a vegetative inoculum of the organism may be prepared by inoculating a small quantity of the culture medium with the spore form of the organism, and the vegetative inoculum obtained may be transferred to the fermentation medium, or, more preferably to one or more seed stages where further growth takes place before transfer to the principal fermentation medium. The fermentation will generally be carried out in the pH range 5.5 to 8.5, preferably 5.5 to 7.5.

Once the compound of formula (II) has been added to the culture, usually with gentle mixing, the cultivation is continued such that the desired product is accumulated. The presence of the product in the fermentation broth may be determined by monitoring extracts of the broth by high performance liquid chromatography, and uv spectroscopy at 238nm.

The product(s) may be isolated from the whole fermentation broth by conventional isolation and separation techniques as described in UK Patent Specifications 2166436 and 2176182.

In a further embodiment of the process according to the invention, the conversion of a compound of

formula (II) into a corresponding 5-OR$^4$ compound of formula (I) may be effected by combining and incubating a compound of formula (II) in a suitable solvent (eg a water miscible organic solvent as previously defined) with a preparation of the enzyme of the invention, desirably in a buffer solution, at, for example, 0° to 60°, preferably 20° to 40° eg about 28°C. The reaction will generally be carried out in the pH range 3.5 to 8.5 eq 5.5 to 7.5. If desired the reaction may be carried out in the presence of a cofactor such as NADH or NADPH. When the reaction is complete, ie when the compound of formula (II) is no longer converted to the compound of the invention (as determined by monitoring extracts of the reaction mixture by high performance liquid chromatography and uv spectroscopy at 238nm) the product is recovered by conventional isolation and separation techniques as described in UK Patent Specifications 2166436 and 2176182.

The enzyme for use in the process of the present invention may be prepared, for example, by culture of a microorganism which produces the enzyme in a nutrient medium. Suitable nutrient media and fermentation conditions for the preparation of the enzyme include those previously described for the preparation of a compound of formula (I) from a compound of formula (II) in the presence of a microorganism. The time at which the required enzymic activity reaches a maximum will, of course, vary according to the microorganism used and, hence, the optimum cultivation time is desirably determined independently for each strain employed.

For microorganisms where the enzyme is extracellular, the liquid culture medium or the filtrate after removal of whole cells may be used as a source of enzyme. Where the enzyme is cell-bound it may be released for use by conventional methods such as sonication, grinding with glass beads, homogenisation, treatment with lytic enzymes or with detergents, after suspension of the cells in a suitable buffer.

The resulting preparation, either with or without removal of cell debris, may be used as a source of enzyme. It is preferred, however, to purify the enzyme further by conventional means. Batch or column chromatography with ion-exchange celluloses or affinity adsorbents or other adsorbents eg hydroxylapatite may conveniently be employed. In addition, the enzyme may be concentrated or further purified by molecular sieve techniques eg ultrafiltration or salting out. In general, during purification procedures, it is desirable to maintain the pH within the range 3 to 11.

The enzyme may be employed in an immobilized form, eg by insolubilisation or entrappment thereof on or in a suitable matrix. Thus an extract of the enzyme may be bound or linked to an otherwise inert inorganic or organic polymer, entrapped on or in a fibre, or on or in a membrane or polymer such as polyacrylamide gel, adsorbed on an ion-exchange resin, crosslinked with a reagent such as glutaraldehyde, or occluded in an envelope such as a bead. Immobilized enzymes may advantageously be employed both in batch processes, after which the enzyme may be reused, and continuous flow processes wherein substrates pass through a column containing the immobilized enzyme.

The group R$^5$ when present in compounds of formula (I) may represent an acyl group e.g. a group of the formula R$^7$CO- or R$^7$OCO- or R$^7$OCS- (where R$^7$ is an aliphatic, araliphatic or aromatic group, for example an alkyl, alkenyl, alkynyl, cycloalkyl, aralkyl or aryl group), a formyl group, a group R$^8$ which is as defined above for R$^7$, a group $R^9SO_2$- (where R$^9$ is a $C_{1-4}$ alkyl or $C_{6-10}$ aryl group), a silyl group, a cyclic or acyclic acetal group, a group $-CO(CH_2)_nCO_2R^{10}$ (where R$^{10}$ is a hydrogen atom or a group as defined above for R$^7$ and n represents zero, 1 or 2) or a group $R^{11}R^{12}NCO$- (where R$^{11}$ and R$^{12}$ may each independently represent a hydrogen atom or a $C_{1-4}$ alkyl group).

Where R$^7$ or R$^8$ are alkyl groups, they may be for example $C_{1-8}$ alkyl groups, e.g. methyl, ethyl, n-propyl, i-propyl, n-butyl, i-butyl, t-butyl or n-heptyl which alkyl groups may also be substituted. Where R$^7$ is a substituted alkyl group it may be substituted by, for example, one or more, eg two or three, halogen atoms (e.g. chlorine or bromine atoms), or a carboxy, $C_{1-4}$ alkoxy (e.g. methoxy, ethoxy), phenoxy or silyloxy group. Where R$^8$ is a substituted alkyl group it may be substituted by a cycloalkyl e.g. cyclopropyl group.

Where R$^7$ and R$^8$ are alkenyl or alkynyl groups, they preferably have 2-8 carbon atoms and where R$^7$ and R$^8$ are cycloalkyl groups, they may be for example $C_{3-12}$ cycloalkyl, such as $C_{3-7}$ cycloalkyl, eg cyclopentyl groups.

Where R$^7$ and R$^8$ are aralkyl groups, they preferably have 1-6 carbon atoms in the alkyl moiety, and the aryl group(s) may be carbocylic or heterocyclic and preferably contain 4-15 carbon atoms e.g. phenyl. Examples of such groups include phen $C_{1-6}$ alkyl eg benzyl groups.

Where R$^7$ and R$^8$ are aryl groups, they may be carbocyclic or heterocyclic and preferably have 4-15 carbon atoms e.g. phenyl.

When R$^5$ is a group $R^9SO_2$-, it may be for example a methylsulphonyl or p-toluenesulphonyl group.

Where R$^5$ represents a cyclic acetal group, it may for example have 5-7 ring members as in the tetrahydropyranyl group.

When $R^5$ represents a silyl group or $R^7$ contains a silyloxy substituent, the silyl group may carry three groups which may be the same or different, selected from alkyl, alkenyl, alkoxy, cycloalkyl, aralkyl, aryl and aryloxy groups. Such groups may be as defined above and particularly include methyl, t-butyl and phenyl groups. Particular examples of such silyl groups are trimethylsilyl and t-butyldimethylsilyl.

When $R^5$ represents a group $-CO(CH_2)_nCO_2R^{10}$, it may for example be a group $-COCO_2R^{10}$ or $-COCH_2CH_2CO_2R^{10}$ where $R^{10}$ represents a hydrogen atom or a $C_{1-4}$ alkyl group (eg methyl or ethyl).

When $R^5$ represents a group $R^{11}R^{12}NCO-$, $R^{11}$ and $R^{12}$ for example may each independently be a hydrogen atom or a methyl or ethyl group.

When $R^6$ represents a $C_{1-8}$ alkyl group it may be for example a methyl, ethyl, n-propyl, i-propyl, n-butyl, i-butyl or t-butyl group, and is preferably a methyl group.

When $R^6$ represents a $C_{3-8}$ alkenyl group it may be for example an allyl group.

Preferred compounds of formula (I) which may be prepared according to the process of the present invention include compounds in which $R^1$ is isopropyl.

Important compounds of formula (I) which may be prepared according to the process of the present invention include those in which $R^2$ represents a hydroxy or ethoxy group and $R^3$ represents a hydrogen atom; or $R^2$ and $R^3$ each represents a hydrogen atom; or $R^2$ and $R^3$ together with the carbon atom to which they are attached represent $>C=O$, $>C=CH_2$ or $>C=NOCH_3$.

Particularly important compounds of formula (I) which may be prepared according to the process of the present invention are those in which:

$R^1$ is isopropyl, $R^2$ and $R^3$ together with the carbon atom to which the are attached represent $>C=NOCH_3$ and $R^4$ is a hydrogen atom;

$R^1$ is isopropyl and $R^2$, $R^3$ and $R^4$ are hydrogen atoms; and

$R^1$ is isopropyl, $R^2$ is hydroxy and $R^3$ and $R^4$ are hydrogen atoms.

Compounds of formula (II) in which $R^1$ is a methyl, ethyl or isopropyl group, $R^2$ is a hydroxyl group and $R^3$ is a hydrogen atom may are described in British Patent Specification 2187742A. The other compounds of formula (II) are described in European Patent Specification 0238258-A1.

The following Preparations and Examples illustrate the invention in which all temperatures are in °C and "L" refers to Litre. In the following Examples hplc (high performance liquid chromatography) was carried out on a column of Spherisorb S5ODS-2 (150mm x 2.1mm) using acetonitrile/water (3:1) as eluant at a rate of 0.5ml/min unless otherwise stated. The column effluent was interfaced with a Finnigan moving belt to a Finnigan MAT4500 mass spectrometer under E.I. conditions for mass spectrum characterisation. The compounds of the following Preparation are named by reference to the known parent 'Factor', Factor A. Factor A (described in UK Patent Specification 2166436) is a compound of formula (I) in which $R^1$ is an isopropyl group, $R^2$ is a hydroxyl group and $R^3$ and $R^4$ are hydrogen atoms.

<u>Intermediate 1</u>

<u>5-Keto Factor A,</u>

prepared as described in Example 2 of British Patent Specification 2187742A.

<u>Intermediate 2</u>

<u>5.23-Diketo Factor A,</u>

prepared as described in Example 2 of EP-A1 238258.

<u>Intermediate 3</u>

<u>5-Keto Factor A 23-ethyl ether,</u>

prepared as described in Example 7 of EP-A1 238258.

<u>Intermediate 4</u>

5-Keto Factor A 23-hemioxalate

5-Keto Factor A (330mg), finely divided calcium carbonate (200mg) and oxalyl chloride (0.07ml) were stirred together in dry dichloromethane (5ml) for 10 min. 2N Hydrochloric acid (4ml) was then added followed 5 min later by ethyl acetate. The organic phase was collected, dried and evaporated and the residue dissolved in diisopropyl ether. Dilution of this solution with petroleum ether (b.p. 40-60°) precipitated the title compound as a white flocculent solid (262mg), $[\alpha]_D^{24}$ +120° (c 0.6, $CHCl_3$),

$$\lambda_{max}^{EtOH}$$

240nm (E′ 425), $\nu$max ($CHBr_3$) 3440 (OH), 1800 ($CO_2H$ dimer), 1720 (ester), and 1676cm$^{-1}$ ($\alpha,\beta$-unsaturated ketone), $\delta$ ($CDCl_3$) include 6.52 (narrow m, 1H), 5.07 (q,3Hz,1H), 3.78 (s,1H), 1.83 (s,3H), and 0.71 (d,7Hz, 3H).

Intermediate 5

5-Keto 23[E]-methoxyimino Factor A,

prepared as described in Example 16 of EP-A1 238258.

Example 1

(a) A spore suspension (0.1ml) of Streptomyces thermoarchaensis NCIB 12213 [prepared as described for Streptomyces thermoarchaensis NCIB 12015 in Example 2 of British Patent Specification 2187742A] was used to inoculate a flask containing the Medium A (2.5ml):

| Medium A | $gL^{-1}$ |
|---|---|
| D-Glucose | 2.5 |
| Malt dextrin MD 30E (Roquette (UK)Ltd) | 25.0 |
| Arkasoy 50 (British Arkady Co.Ltd) | 12.5 |
| Molasses | 1.5 |
| $K_2HPO_4$ | 0.125 |
| Calcium carbonate | 1.25 |
| MOPS (3-(N-morpholino)propanesulphonic acid) | 21.0 |
| [Distilled water to 1 L, pH adjusted to 6.5 with 5N NaOH before autoclaving]. | |

This culture was incubated at 31° for 4 days on a rotary shaker operating at 250 rev/min, at which time Intermediate 1 (1mg) in dimethylsulphoxide (0.05ml) was added. The culture was incubated for a further 24h at 31°, then centrifuged (approx. 10000 rpm/10 min), the supernatant removed and (1.8ml) of methanol added to the residue. The resulting suspension was allowed to stand for 1h at room temperature and then centrifuged. The supernatant was analysed by hplc which showed by comparison with an authentic sample described in UK Patent Specification 2166436, the presence in the test sample of the compound of formula (I) where $R^1$ is an isopropyl group, $R^2$ is a hydroxyl group, $R^3$ is a hydrogen atom and $R^4$ is a hydrogen atom [3.3% - expressed as % of the starting material added that was converted to the product].

(b) A repeat of the part (a) experiment with Streptomyces avermitilis ATCC 31780 and Intermediate 1 yielded an extract which on hplc analysis showed the presence of the compound of formula (I) in which $R^1$ is an isopropyl group, $R^2$ is a hydroxyl group, $R^3$ is a hydrogen atom and $R^4$ is a hydrogen atom [8.6% - % expressed as in part (a)].

(c) In a similar manner to part (a) Intermediate 1 was incubated with Streptomyces thermoarchaensis NCIB 12212, except that the culture was prepared from a spore suspension added to Medium A but which

after 3 days incubation as described had been used (0.1ml transferred) to inoculate a fresh portion of the same medium (2.5ml). Intermediate 1 (1mg in 0.05ml dimethylsulphoxide) was added to this later culture 2 days after and the culture was then incubated for a further two days before being harvested, extracted and analysed as described in part (a). Hplc analysis of a test sample showed the presence of the compound of formula (I) in which $R^1$ is an isopropyl group, $R^2$ is a hydroxyl group, $R^3$ is a hydrogen atom and $R^4$ is a hydrogen atom [5.1% - % expressed as in part (a)].

Example 2

(a) A spore suspension (0.4ml) of Streptomyces thermoarchaensis NCIB 12213 [prepared as described for Example 1] was used to inoculate a 250ml shake flask containing Medium A (25ml). This culture was incubated at 28° for 2 days on a rotary shaker operated at 250rev/min. After 2 days a portion (5ml) of this culture was added to a 50ml shake flask and a 20mg/ml solution of Intermediate 2 in methanol (50µl) was added. The culture was incubated for a further 2-3 days under the same conditions, then treated with an equal volume of methanol. The resulting suspension was shaken for 1h and centrifuged. The supernatant was analysed by hplc/mass spectrometry. A peak with a retention time 3 min. had a mass spectrum characteristic of the compound of formula (I) in which $R^1$ is isopropyl, $R^2$ and $R^3$ together with the carbon atom to which they are attached represent >C=O and $R^4$ is a hydrogen atom by comparison with an authentic sample described in UK Patent Specification 2176182.

(b) A repeat of the part (a) experiment with Streptomyces thermoarchaensis NCIB 12213 and Intermeidate 3 yielded an extract which on hplc/mass spectrometry analysis (using acetonitrile/water 9:1 as eluant) showed the presence of a compound of formula (I) in which $R^1$ is an isopropyl group, $R^2$ is an ethoxy group, $R^3$ is a hydrogen atom and $R^4$ is a hydrogen atom by comparison with an authentic sample described in UK Patent Specification 2176182.

(c) A repeat of the part (a) experiment with Streptomyces thermoarchaensis NCIB 12213 and Intermediate 4 yielded an extract which on hplc analysis showed the presence of a compound of formula (I) in which $R^1$ is an isopropyl group, $R^2$ is a group $-OCOCO_2H$, $R^3$ is a hydrogen atom and $R^4$ is a hydrogen atom by comparison with an authentic sample described in UK Patent Specification 2176182.

(d) A repeat of the part (a) experiment with Streptomyces thermoarchaensis NCIB 12213 and Intermediate 5 yielded an extract which an hplc analysis showed the presence of a compound of formula (I) in which $R^1$ is an isopropyl group, $R^2$ and $R^3$ together with the carbon atom to which they are attached represent $>C=NOCH_3$ and $R^4$ is a hydrogen atom by comparison with an authentic sample described in UK Patent Specification 2192630A.

E.I. mass spectroscopy of a sample isolated from analytical hplc yielded a molecular ion at 639 and gave characteristic fragments at 621, 527, 511, 496, 448, 434, 368, 264, 248 and 151.

## Claims

1. A process for the production of compounds of formula (I)

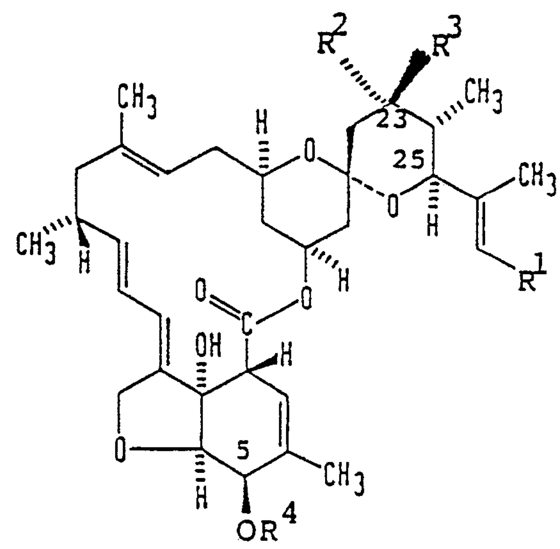

(I)

[wherein $R^1$ is a methyl, ethyl or isopropyl group; $R^2$ is a hydrogen atom or a group $OR^5$ (where $OR^5$ is a hydroxyl group or a substituted hydroxyl group having up to 25 carbon atoms) and $R^3$ is a hydrogen atom, or $R^2$ and $R^3$ together with the carbon atom to which they are attached represent >C=O, >C=$CH_2$ or >C=$NOR^6$ (where $R^6$ represents a hydrogen atom, $C_{1-8}$ alkyl group or a $C_{3-8}$ alkenyl group and the group >C=$NOR^6$ is in the E configuration); and $R^4$ is a hydrogen atom or a methyl group] which comprises incubating a compound of formula (II)

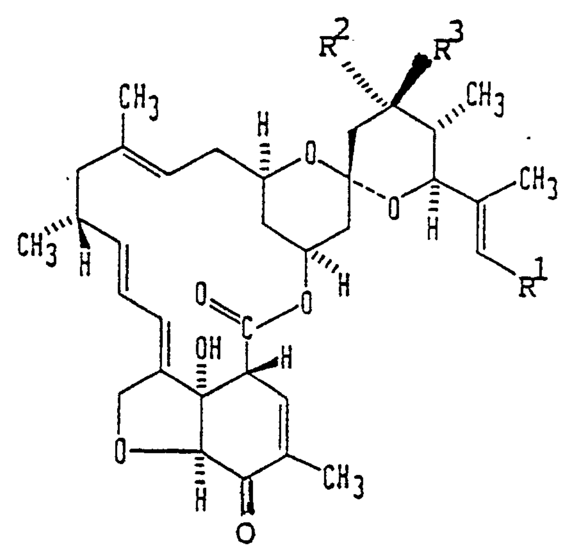

(II)

(wherein $R^1$, $R^2$ and $R^3$ are as defined above) in a suitable medium in the presence of a microorganism or an enzyme derived therefrom or a preparation derived from a microorganism containing the enzyme of interest capable of effecting the conversion .

2. A process according to claim 1 in which the microorganism is a strain of Streptomyces thermoarchaensis, Streptomyces cyaneogriseus noncyanogenus, Streptomyces avermitilis or Streptomyces hygroscopicus subsp. aureolacrimosus.

3. A process according to claim 1 in which the microorganism is Streptomyces thermoarchaensis NCIB 12212 and 12213 and mutants of these strains, Streptomyces avermitilis ATCC 31272 or 31780 or Streptomyces hygroscopicus subsp. aureolacrimosus FERM P 1438.

4. A process according to claim 1 which comprises feeding the compound of formula (II) in a suitable solvent into a fermentation medium comprising the microorganism in the presence of assimilable sources of carbon, nitrogen and mineral salts.

5. A process according to claim 1 which is carried out at a pH of 5.5 to 7.5 and a temperature of 25 to 40 °C.

6. A process according to claim 1 in which $R^1$ in formula (I) and formula (II) is an isopropyl group.

7. A process according to claim 1 in which, in formula (I) and formula (II), $R^2$ is a hydroxy or ethoxy group and $R^3$ is a hydrogen atom; or $R^2$ and $R^3$ each represents a hydrogen atom; or $R^2$ and $R^3$ together with the carbon atom to which they are attached represent $>C=0$, $>C=CH_2$ or $>C=NOCH_3$.

8. A process according to claim 1 in which, in formula (I):

$R^1$ is isopropyl, $R^2$ and $R^3$ together with the carbon atom to which they are attached represent $>C=NOCH_3$ and $R^4$ is a hydrogen atom;

$R^1$ is isopropyl, $R^2$, $R^3$ and $R^4$ are hydrogen atoms; or

$R^1$ is isopropyl, $R^2$ is hydroxy and $R^3$ and $R^4$ are hydrogen atoms.